# FASCICULE DE BREVET EUROPEEN

(11) **EP 0 321 950 B1**
(45) Date de publication et mention de la délivrance du brevet: **12.01.1994**
(21) Numéro de dépôt: 88121399.5
(22) Date de dépôt: 21.12.1988
(51) Int. Cl.: A61K 7/06, A61K 31/505

(54) **Association de dérivés de pyrimidine et d'hydrocortisone pour induire et stimuler la croissance des cheveux et diminuer leur chute**
Assoziat, bestehend aus Pyrimidinderivaten und Hydrocortison, zum Induzieren und Stimulieren des Haarwuchses und zum Vermindern des Haarausfalls
Combination of pyrimidine derivatives and hydrocortisone to induce and stimulate the hair growth and to reduce hair loss

(30) Priorité: 22.12.1987 LU 87091
(43) Date de publication de la demande: 28.06.1989
(73) Titulaire: L'OREAL, 75008 Paris (FR)
(72) Inventeur: Grollier, Jean-François, F-75004 Paris (FR); Rosenbaum, Georges, F-92600 Asnières (FR)
(74) Mandataire: Casalonga, Axel

(56) Documents cités:
- WO-A- 8/87361
- WO-A-85/05270
- STN INTERNATIONAL, File Supplier, Karlsruhe (DE)#
- PATENT ABSTRACTS OF JAPAN, vol. 11, no. 3 (C-395)[2450]; p. 107 C395#
- PATENT ABSTRACTS OF JAPAN, vol. 11, no. 115 (C-415)[2562]; p. 145 C 415#

## Description

L'invention est relative à l'association de dérivés de pyrimidine et d'hydrocortisone en vue d'induire et de stimuler la croissance des cheveux et diminuer leur chute.

L'activité des follicules pileux est cyclique. A la phase anagène active, qui dure plusieurs années et au cours de laquelle le cheveu s'allonge, succède une phase de repos (télogène) de quelques mois. A la fin de cette période de repos, le cheveu tombe et un autre cycle recommence.

La chevelure se renouvelle donc en permanence; sur les 100.000 à 150.000 cheveux que comporte une chevelure, à chaque instant 10% environ sont au repos et seront donc remplacés en quelques mois.

Dans presque tous les cas, la chute des cheveux survient chez des sujets prédisposés génétiquement; elle atteint plus particulièrement les hommes.

Cette alopécie est une perturbation du renouvellement capillaire qui entraîne, dans un premier temps, une accélération de la fréquence des cycles aux dépens de la qualité des cheveux puis de leur quantité. Il y a un appauvrissement progressif de la chevelure par régression d'une partie des cheveux dits "terminaux" au stade de "duvets". Des zones sont touchées préférentiellement : golfes temporaux-frontaux, ... chez les hommes; alopécie diffuse du vertex chez les femmes.

Parmi les alopécies, les alopécies acquises, non cicatricielles et diffuses, telle que l'alopécie androgénétique ou androgénique, sont particulièrement difficiles à traiter par opposition aux alopécies localisées telle que la pelade qui serait d'origine immunologique.

On a déjà proposé, dans le passé, le traitement topique de la pelade avec des corticostéroïdes fluorés tels que la fluocinolone, l'halcinonide et la dexaméthasone. Par contre, jusqu'à présent, leur utilisation dans le domaine du traitement de l'alopécie androgénétique ne s'est pas révélée satisfaisante.

On a par ailleurs également utilisé des composés tels que l'amino-6 dihydro-1,2 hydroxy-1 imino-2 pipéridino-4 pyrimidine ou "Minoxidil" dans des compositions permettant de réduire ou de supprimer l'effet de l'alopécie et d'induire et de stimuler la croissance des cheveux et diminuer leur chute.

Le document WO 88/07361 décrit une association contenant dans un milieu hydroalcoolique de l'hydrocortisone et du "Minoxidil" pour le traitement de l'alopécie
La demanderesse a découvert maintenant, qu'en associant de l'hydrocortisone avec certains dérivés de pyrimidine en milieu anhydre, on constatait de façon surprenante, une induction et une stimulation améliorées de la croissance des cheveux et une action plus forte sur le freinage de la chute des cheveux, en particulier dans le cas des alopécies diffuses.

Cette efficacité se caractérise en particulier par une activité supérieure par rapport aux dérivés de pyrimidine utilisés seuls ou par rapport aux agents anti-inflammatoires stéroïdiens.

La demanderesse a également constaté que l'association conforme à l'invention permettait d'obtenir un effet plus rapide que par le passé, ou d'utiliser le dérivé de pyrimidine à une concentration plus faible.

Afin de déterminer l'efficacité ou la rapidité d'action des compositions de l'alopécie, on utilise généralement le trichogramme et plus particulièrement le photo-trichogramme permettant de déterminer entre autre le pourcentage de cheveux en phase anagène par rapport aux cheveux en phase télogène.

L'invention cherche donc essentiellement à augmenter le rapport du nombre de cheveux en phase anagène par rapport au nombre de cheveux en phase télogène.

Un objet de l'invention est donc constitué par l'association d'hydrocortisone avec des dérivés de pyrimidine en milieu anhydre, en vue d'induire ou de stimuler la croissance des cheveux et diminuer leur chute.

Un autre objet de l'invention est constitué par une composition cosmétique ou pharmaceutique anhydre contenant cette association.

L'invention a également pour objet un dispositif à plusieurs compartiments ou "kits" ou nécessaire, comportant l'association.

D'autres objets de l'invention apparaîtront à la lecture de la description et des exemples qui suivent.

L'association conforme à l'invention est essentiellement caractérisée par le fait qu'elle est constituée par :
a/ un composant (A) comprenant, dans un milieu physiologiquement acceptable anhydre, au moins de l'hydrocortisone ou ses sels et esters,
b/ un composant (B) contenant dans un milieu physiologiquement acceptable anhydre, au moins un dérivé de pyrimidine, répondant à la formule : 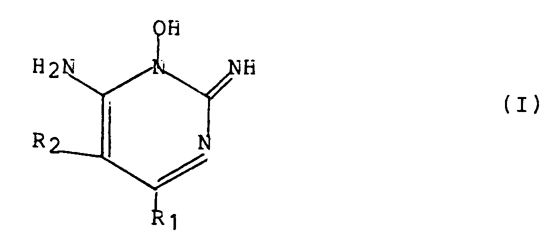 dans laquelle R₁ désigne un groupement 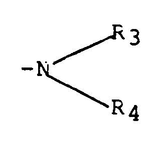 dans lequel R₃ et R₄, indépendamment l'un de l'autre, désignent hydrogène, un groupement alkyle, alcényle, alkylaryle, cycloalkyle, R₃ et R₄ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement R₂ est choisi parmi un atome d'hydrogène, un groupement alkyle, alcényle, alkylalcoxy, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps en vue d'induire de stimuler la croissance des cheveux et diminuer leur chute.

Les sels ou esters d'hydrocortisone sont choisis en particulier parmi le 21-acétate, le 21-bendazac, le 17-valérate, le 17-butyrate, le 21-cyclopentylpropionate, le 21-hémisuccinate, l'hexanoate, le 21-phosphate disodique, le 21-succinate sodique, le terbutylacétate.

L'hydrocortisone et l'hydrocortisone 21-acétate sont particulièrement préférés.

Pour les composés de formule (I), le groupement alkyle ou alcoxy désigne de préférence un groupement ayant 1 à 4 atomes de carbone; le groupement alcényle désigne de préférence un groupement ayant 2 à 5 atomes de carbone; le groupement aryle désigne de préférence un groupement phényle et le groupement cycloalkyle désigne de préférence un groupement ayant 4 à 6 atomes de carbone.

Les composés particulièrement préférés de formule (I) sont choisis parmi les composés dans lesquels R₂ désigne hydrogène et R₁ désigne un groupement : 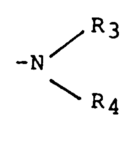
dans lequel R₃ et R₄ forment un cycle pipéridinyle, ainsi que leurs sels tels que, par exemple, le sulfate.

Parmi ces composés, un composé particulièrement préféré est constitué par l'amino-6 dihydro-1,2 hydroxy-1 imino-2 pipéridino-4 pyrimidine, encore appelé "Minoxidil".

L'hydrocortisone ou ses sels et esters est utilisé dans le composant (A) dans des proportions comprises entre 0,01 et 5% en poids, de préférence entre 0,02 et 3%, et plus particulièrement entre 0,02 et 1% en poids; le dérivé de pyrimidine de formule (I) est utilisé de préférence dans le composant (B) dans des proportions comprises entre 0,05 et 10% en poids, de préférence entre 0,05 et 5% en poids et en particulier entre 0,5 et 4% en poids.

Lorsque les composants (A) et (B) sont utilisés dans une composition unique, la proportion en hydrocortisone, ses sels ou esters est comprise entre 0,01 et 3% en poids par rapport au poids total de la composition, de préférence entre 0,01 et 2% et en particulier entre 0,01 et 1%, et le dérivé de pyrimidine de formule (I) est utilisé dans des proportions comprises entre 0,05 et 6% en poids par rapport au poids total de la composition, de préférence entre 0,1 et 5% et en particulier entre 0,5 et 3% en poids.

Le milieu physiologiquement acceptable pour les composants (A) et (B) est un milieu anhydre utilisable en pharmacie et en cosmétique et peut être constitué notamment par un solvant ou un mélange de solvants organiques cosmétiquement ou pharmaceutiquement acceptables.

Les solvants utilisables sont choisis plus particulièrement parmi les alcools inférieurs en C₁-C₄ tels que l'alcool éthylique, l'alcool isopropylique, l'alcool tertiobutylique, des alkylèneglycols comme le propylèneglycol, des alkyléthers de mono- et de dialkylèneglycol, et plus particulièrement le monoéthyléther d'éthylèneglycol, le monométhyléther de propylèneglycol et le monoéthyléther de diéthylèneglycol.

On appelle milieu anhydre un milieu contenant moins de 1% d'eau.

Le milieu physiologiquement acceptable peut être épaissi ou non. On utilise dans ce but des agents épaississants ou gélifiants bien connus dans l'état de la technique, tels que plus particulièrement les hétérobiopolysaccharides tels que la gomme de xanthane ou les scléroglucanes, les dérivés de cellulose, les polymères acryliques réticulés ou non.

Les épaississants sont présents de préférence dans des proportions comprises entre 0,1 et 5% en poids et en particulier entre 0,4 et 3% en poids, par rapport au poids total de chacun des composants lorsqu'ils sont utilisés de façon séparée ou par rapport au poids total de la composition contenant les composants (A) et (B).

Les compositions constituées, soit par les composants (A) et (B) ou par la composition contenant les deux composants (A) et (B), peuvent également contenir tous autres adjuvants habituellement utilisés dans les compositions destinées à une application topique, cosmétique ou pharmaceutique, et plus particulièrement des agents conservateurs, des agents complexants, des colorants, des agents alcalinisants ou acidifiants, des agents tensio-actifs, anioniques, cationiques, non ioniques, amphotères ou leurs mélanges, des polymères anioniques, cationiques, non ioniques ou amphotères, ainsi que leurs mélanges.

Ces compositions peuvent également être conditionnées sous pression dans des dispositifs aérosols.

Dans le composant (B), les dérivés de pyrimidine de formule (I) peuvent être présents, soit sous forme dissoute dans le milieu physiologiquement acceptable, ou alors en totalité ou partiellement en suspension dans ce milieu, en particulier sous forme de particules ayant une granulométrie inférieure à 80 microns, de préférence inférieure à 20 µm et en particulier inférieure à 5 µm.

Une première forme de réalisation de l'invention consiste à utiliser l'association définie ci-dessus, sous forme d'une composition contenant les composants (A) et (B).

Une forme particulièrement préférée de l'invention consiste à conserver dans des dispositifs séparés les composants (A) et (B) et à préparer la composition contenant l'hydrocortisone, ses sels ou esters et le dérivé de pyrimidine de formule (I) de façon extemporanée tout juste avant application.

Une autre forme de réalisation consiste enfin à appliquer les composants (A) et (B) de façon séparée, soit simultanément, soit de façon successive ou décalée dans le temps.

L'association conforme à l'invention peut être conditionnée dans ces cas, en particulier dans un dispositif à plusieurs compartiments encore appelé "Kit" ou nécessaire, le premier compartiment contient le composant (A) avec l'hydrocortisone, ses sels ou esters et le second compartiment contient le composant (B) à base du dérivé de pyrimidine de formule (I). Ce dispositif peut être équipé d'un moyen de mélange des compositions tout juste au moment de l'application.

Le traitement pour induire et stimuler la croissance des cheveux et diminuer leur chute consiste principalement à appliquer sur les zones alopéciques du cuir chevelu et des cheveux d'un individu, l'association telle que définie ci-dessus, soit au moyen d'une composition unique, soit par application des composants (A) et (B) ou (B) et (A), de façon successive ou décalée dans le temps.

Le mode d'application préféré consiste à appliquer 1 à 2 grammes de la composition ou de chacun des composants (A) et (B) sur la zone alopécique du cuir chevelu à une fréquence de une à deux applications par jour pendant 1 à 7 jours par semaine et ceci pendant une durée de 1 à 6 mois.

La demanderesse a constaté des effets particulièrement significatifs au bout de deux mois de traitement.

Le procédé de traitement présente les caractéristiques d'un traitement thérapeutique dans la mesure où l'association conforme à l'invention a une activité thérapeutique au niveau des mécanismes biologiques du cycle pilaire et des dysfonctionnements de celui-ci.

Un objet de l'invention est donc également l'utilisation de l'association telle que définie ci-dessus pour la préparation d'un médicament ayant pour effet d'induire ou de stimuler la croissance des cheveux et de freiner leur chute.

Le procédé présente, par ailleurs, les caractéristiques d'un procédé cosmétique dans la mesure où il permet d'embellir les cheveux ou le cuir chevelu au sens cosmétique du terme.

Les exemples suivants sont destinés à illustrer l'invention sans pour autant présenter un caractère limitatif.

### EXEMPLE 1

On prépare la composition suivante :

| | |
|---|---|
| - Minoxidil | 0,625 g |
| - Hydrocortisone | 0,625 g |
| - Propylèneglycol distillé | 6,45 g |
| - Alcool éthylique absolu | qsp 100,00 g |

On applique cette lotion quotiennement sur les zones alopéciques du cuir chevelu pendant une durée de traitement de 3 mois. Chaque mois, on constate un effet repousse des cheveux par une augmentation de leur densité totale et de la densité des cheveux en phase anagène.

### EXEMPLE 2

On prépare la composition suivante :

| | |
|---|---|
| - Minoxidil | 2,5 g |
| - Hydrocortisone | 0,625 g |
| - Propylèneglycol distillé | 6,45 g |
| - Alcool éthylique absolu | qsp 100,00 g |

### EXEMPLE 3

On conditionne en kit deux compositions (A) et (B) renfermant respectivement :

| Composition (A) | |
|---|---|
| - Hydrocortisone | 2,00 g |
| - Propylèneglycol distillé | 6,45 g |
| - Alcool éthylique absolu | qsp 100,00 g |

| Composition (B) | |
|---|---|
| - Minoxidil | 2,5 g |
| - Propylèneglycol distillé | 6,45 g |
| - Alcool éthylique absolu | qsp 100,00 g |

### EXEMPLE 4

| | |
|---|---|
| - Minoxidil | 2,00 g |
| - Hydrocortisone 21-acétate | 0,30 g |
| - Propylèneglycol distillé | 6,45 g |
| - Alcool éthylique absolu | qsp 100,00 g |

### EXEMPLE 5

| | |
|---|---|
| - Minoxidil | 1,5 g |
| - Hydrocortisone 21-acétate | 0,45 g |
| - Monométhyléther du propylène glycol | 50,00 g |
| - Alcool éthylique absolu | qsp 100,00 g |

### EXEMPLE 6

On conditionne en kit deux compositions (A) et (B) renfermant respectivement :

| Composition (A) | |
|---|---|
| - Hydrocortisone 21-hémisuccinate | 0,3 g |
| - Alcool éthylique absolu | qsp 100,00 g |

| Composition (B) | |
|---|---|
| - Minoxidil | 2,5 g |
| - Propylèneglycol distillé | 6,45 g |
| - Alcool éthylique absolu | qsp 100,00 g |

On applique (A) puis (B) en succession immédiate sur les zones alopéciques du cuir chevelu dont on mélange 25% en poids de la composition (A) avec 75% en poids de la composition (B) avant l'application.

## Revendications (Revendications pour l'(les) Etat(s) contractant(s) suivant(s): AT, BE, CH, DE, FR, GB, IT, LI NL, SE)

1. Association destinée à induire et à stimuler la croissance des cheveux et diminuer leur chute, caractérisée par le fait qu'elle comprend :
a/ un composant (A) contenant dans un milieu physiologiquement acceptable anhydre, au moins de l'hydrocortisone, ses sels ou esters, et
b/ un composant (B) contenant dans un milieu physiologiquement acceptable anhydre au moins un dérivé de pyrimidine, répondant à la formule : 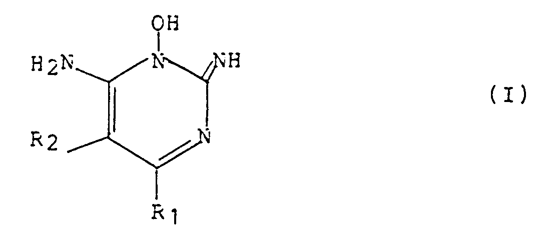 dans laquelle R₁ désigne un groupement 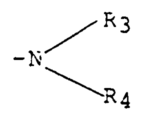 dans lequel R₃ et R₄, indépendamment l'un de l'autre, désignent hydrogène, un groupement alkyle, alcényle, alkylaryle, cycloalkyle, R₃ et R₄ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement R₂ est choisi parmi un atome d'hydrogène, un groupement alkyle, alcényle, alcoxyalkyl, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, sur le cuir chevelu ou les cheveux.

2. Association selon la revendication 1, caractérisée par le fait que les sels ou esters d'hydrocortisone sont choisis parmi le 21-acétate, le 21-bendazac, le 17-valérate, le 17-butyrate, le 21-cyclopentylpropionate, le 21-hémisuccinate, l'hexanoate, le 21-phosphate disodique, le 21-succinate sodique, le terbutylacétate.

3. Association selon les revendications 1 ou 2, caractérisée par le fait que le dérivé de pyrimidine de formule (I) est l'amino-6 dihydro-1,2 hydroxy-1 imino-2 pipéridino-4 pyrimidine ou "Minoxidil".

4. Association selon l'une quelconque des revendications 1 à 3, caractérisée par le fait que l'hydrocortisone, ses sels ou esters sont présents dans le composant (A) dans des proportions comprises entre 0,01 et 5% en poids, de préférence entre 0,02 et 3% et en particulier entre 0,02 et 1% en poids, et que le dérivé de pyrimidine est présent dans le composant (B) dans des proportions comprises entre 0,05 et 10% en poids, de préférence entre 0,05 et 5% en poids et en particulier entre 0,5 et 4% en poids.

5. Association selon l `une quelconque des revendications 1 à 4, caractérisée par le fait que l'association est présente dans une composition unique, dans laquelle l'hydrocortisone, ses sels ou esters sont présents dans des proportions comprises entre 0,01 et 3% en poids par rapport au poids total de la composition, de préférence entre 0,01 et 2% et en particulier entre 0,01 et 1%, et que le dérivé de pyrimidine de formule (I) est utilisé dans des proportions comprises entre 0,05 et 6% en poids par rapport au poids total de la composition, de préférence entre 0,1 et 5% et en particulier entre 0,5 et 3% en poids.

6. Association selon l'une quelconque des revendications 1 à 5, caractérisée par le fait que le milieu physiologiquement acceptable anhydre pour les composants (A) et (B) est un milieu constitué par un mélange de solvants organiques acceptables en cosmétique ou en pharmacie choisis parmi les alcools inférieurs en C₁-C₄, les alkylène-glycols, les alkyléthers de mono- et de dialkylène-glycol.

7. Association selon l'une quelconque des revendications 1 à 6, caractérisée par le fait que l'un au moins des composants (A) ou (B) est épaissi au moyen d'agents épaississants ou gélifiants.

8. Association selon l'une quelconque des revendications 1 à 7, caractérisée par le fait que l'un au moins des composants (A) ou (B) contient également des adjuvants cosmétiquement ou pharmaceutiquement acceptables, choisis parmi des agents conservateurs, des agents complexants, des colorants, des agents alcalinisants ou acidifiants, des agents tensio-actifs, anioniques, cationiques, non ioniques ou amphotères, ainsi que leurs mélanges, des polymères anioniques, cationiques, non ioniques ou amphotères, ainsi que leurs mélanges.

9. Dispositif à plusieurs compartiments ou "kit", caractérisé par le fait qu'il comprend dans un premier compartiment un composant (A) tel que défini dans l'une quelconque des revendications 1 à 8, et dans un second compartiment le composant (B) tel que défini également dans l'une quelconque des revendications 1 à 9.

10. Association selon l'une quelconque des revendications 1 à 8, pour son application comme médicament pour le traitement thérapeutique de la chute des cheveux.

11. Utilisation de l'association selon l'une quelconque des revendications 1 à 8, pour la préparation d'un médicament destiné à être utilisé dans le traitement thérapeutique de l'alopécie androgénétique.

## Revendications (Revendications pour l'(les) Etat(s) contractant(s) suivant(s): GR)

1. Association destinée à induire et à stimuler la croissance des cheveux et diminuer leur chute, caractérisée par le fait qu'elle comprend :
a/ un composant (A) contenant dans un milieu physiologiquement acceptable anhydre, au moins de l'hydrocortisone, ses sels ou esters, et
b/ un composant (B) contenant dans un milieu physiologiquement acceptable anhydre au moins un dérivé de pyrimidine, répondant à la formule : 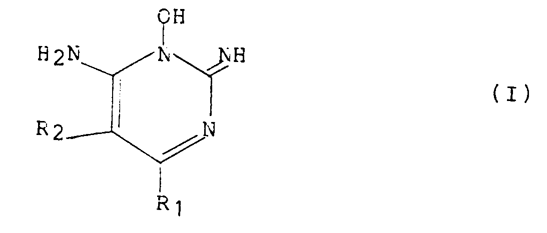 dans laquelle R₁ désigne un groupement 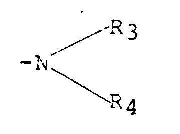 dans lequel R₃ et R₄, indépendamment l'un de l'autre, désignent hydrogène, un groupement alkyle, alcényle, alkylaryle, cycloalkyle, R₃ et R₄ peuvent également former un hétérocycle avec l'atome d'azote auquel ils sont liés' choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement R₂ est choisi parmi un atome d'hydrogène, un groupement alkyle, alcényle, alcoxyalkyl, cycloalkyle, aryle, alkylaryle, arylalkyle, alkylarylalkyle, alcoxyarylalkyle ou haloarylalkyle, ainsi que les sels d'addition d'acides physiologiquement acceptables, les composants (A) et (B) faisant partie d'une même composition ou étant destinés à être utilisés séparément, soit simultanément, soit de façon successive ou décalée dans le temps, sur le cuir chevelu ou les cheveux.

2. Association selon la revendication 1, caractérisée par le fait que les sels ou esters d'hydrocortisone sont choisis parmi le 21-acétate, le 21-bendazac, le 17-valérate, le 17-butyrate, le 21-cyclopentylpropionate, le 21-hémisuccinate, l'hexanoate, le 21-phosphate disodique, le 21-succinate sodique, le terbutylacétate.

3. Association selon les revendications 1 ou 2, caractérisée par le fait que le dérivé de pyrimidine de formule (I) est l'amino-6 dihydro-1,2 hydroxy-1 imino-2 pipéridino-4 pyrimidine ou "Minoxidil".

4. Association selon l'une quelconque des revendications 1 à 3, caractérisée par le fait que l'hydrocortisone, ses sels ou esters sont présents dans le composant (A) dans des proportions comprises entre 0,01 et 5% en poids, de préférence entre 0,02 et 3% et en particulier entre 0,02 et 1% en poids, et que le dérivé de pyrimidine est présent dans le composant (B) dans des proportions comprises entre 0,05 et 10% en poids, de préférence entre 0,05 et 5% en poids et en particulier entre 0,5 et 4% en poids.

5. Association selon l'une quelconque des revendications 1 à 4, caractérisée par le fait que l'association est présente dans une composition unique, dans laquelle l'hydrocortisone, ses sels ou esters sont présents dans des proportions comprises entre 0,01 et 3% en poids par rapport au poids total de la composition, de préférence entre 0,01 et 2% et en particulier entre 0,01 et 1%, et que le dérivé de pyrimidine de formule (I) est utilisé dans des proportions comprises entre 0,05 et 6% en poids par rapport au poids total de la composition, de préférence entre 0,1 et 5% et en particulier entre 0,5 et 3% en poids.

6. Association selon l'une quelconque des revendications 1 à 5, caractérisée par le fait que le milieu physiologiquement acceptable anhydre pour les composants (A) et (B) est un milieu constitué par un mélange de solvants organiques acceptables en cosmétique ou en pharmacie choisis parmi les alcools inférieurs en C₁-C₄, les alkylène-glycols, les alkyléthers de mono- et de dialkylène-glycol.

7. Association selon l'une quelconque des revendications 1 à 6, caractérisée par le fait que l'un au moins des composants (A) ou (B) est épaissi au moyen d'agents épaississants ou gélifiants.

8. Association selon l'une quelconque des revendications 1 à 7, caractérisée par le fait que l'un au moins des composants (A) ou (B) contient également des adjuvants cosmétiquement ou pharmaceutiquement acceptables, choisis parmi des agents conservateurs, des agents complexants, des colorants, des agents alcalinisants ou acidifiants, des agents tensio-actifs, anioniques, cationiques, non ioniques ou amphotères, ainsi que leurs mélanges, des polymères anioniques, cationiques, non ioniques ou amphotères, ainsi que leurs mélanges.

9. Dispositif à plusieurs compartiments ou "kit", caractérisé par le fait qu'il comprend dans un premier compartiment un composant (A) tel que défini dans l'une quelconque des revendications 1 à 8, et dans un second compartiment le composant (B) tel que défini également dans l'une quelconque des revendications 1 à 9.

10. Procédé de préparation d'une association à deux composants (A) et (B) destinées à induire et à stimuler la croissance des cheveux et diminuer leure chute, caractérisé par le fait que
a) on prépare un composant (A) en introduisant dans un milieu physiologiquement acceptable anhydre, au moins de l'hydrocortisone, ses sels ou esters et
b) on prépare un composant (B) en introduisant dans un milieu physiologiquement acceptable anhydre au moins un dérivé de pyrimidine, répondant à la formule : 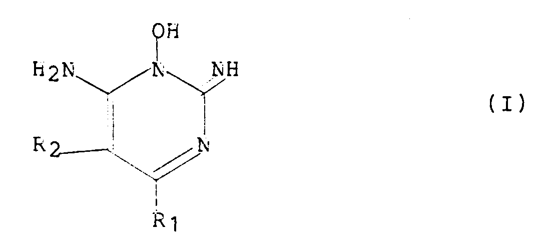 dans laquelle R₁ désigne un groupement 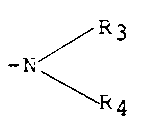 dans lequel R₃ et R₄, indépendamment l'un de l'autre, désignent hydrogène, un groupement alkyle, alcényle, alkylaryle, cycloalkyle, R₃ et R₄ peuvent également former un hétérocycle avec l'atome d'azote auquel ils sont liés, choisi parmi les groupements aziridinyle, azétidinyle, pyrrolidinyle, pipéridinyle, hexahydroazépinyle, heptaméthylèneimine, octaméthylèneimine, morpholine et alkyl(inférieur)-4 pipérazidinyle, les groupements hétérocycliques pouvant être substitués sur les atomes de carbone par un à trois groupements alkyle inférieur, hydroxy ou alcoxy; le groupement R₂ est choisi parmi un atome d'hydrogène, un groupement alkyle, alcényle, alcoxyalkyl, cycloalkyle, aryle, alkylaryle, arylalkyle, alkylarylalkyle, alcoxyarylalkyle ou haloarylalkyle, ainsi que les sels d'addition d'acides physiologiquement acceptables ;
les composants (A) et (B) étant soit mélangés soit stockés séparément, en vue de leur application directe ou de façon successive ou décalée dans le temps, sur le cuir chevelu et les cheveux.

## Claims (Claims for the following Contracting State(s): AT, BE, CH, DE FR, GB, IT, LI, NL, SE)

1. Combination intended to induce and to stimulate the growth of hair and to reduce its loss, characterized in that it comprises:
a) a component (A) containing, in an anhydrous, physiologically acceptable medium, at least hydrocortisone, its salts or esters, and
b) a component (B) containing, in an anhydrous, physiologically acceptable medium, at least one pyrimidine derivative corresponding to the formula: 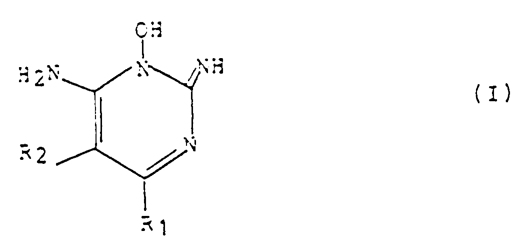 in which R₁ denotes a group 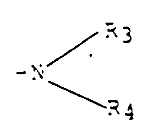 in which R₃ and R₄, independently of each other, denote hydrogen or an alkyl, alkenyl, alkylaryl or cycloalkyl group; R₃ and R₄ may also form a heterocyclic ring with the nitrogen atom to which they are linked, which is chosen from aziridinyl, azetidinyl, pyrrolidinyl, piperidyl, hexahydroazepinyl, heptamethyleneimine, octamethyleneimine, morpholine and 4-(lower)alkylpiperazidinyl groups, it being possible for the heterocyclic groups to be substituted on the carbon atoms by one to three lower alkyl, hydroxyl or alkoxy groups; the group R₂ is chosen from a hydrogen atom and an alkyl, alkenyl, alkylalkoxy, cycloalkyl, aryl, alkylaryl, arylalkyl, alkylarylalkyl, alkoxyarylalkyl or haloarylalkyl group, and the addition salts of physiologically acceptable acids, the components. (A) and (B) forming part of the same single composition or being intended to be employed separately, either simultaneously or successively or after an interval of time, on the scalp or the hair.

2. Combination according to Claim 1, characterized in that the hydrocortisone salts or esters are chosen from the 21-acetate, the 21-bendazac, the 17-valerate, the 17-butyrate, the 21-cyclopentylpropionate, the 21-hemisuccinate, the hexanoate, the disodium 21-phosphate, the sodium 21-succinate and the tert-butylacetate.

3. Combination according to Claims 1 or 2, characterized in that the pyrimidine derivative of formula (I) is 6-amino-1,2-dihydro-1-hydroxy-2-imino-4-piperidino-pyrimidine or "Minoxidil".

4. Combination according to any one of Claims 1 to 3, characterized in that hydrocortisone, its salts or esters are present in component (A) in proportions of between 0.01 and 5 % by weight, preferably between 0.02 and 3 % and in particular between 0.02 and 1 % by weight, and that the pyrimidine derivative is present in component (B) in proportions of between 0.05 and 10 % by weight, preferably between 0.05 and 5 % by weight and in particular between 0.5 and 4 % by weight.

5. Combination according to any one of Claims 1 to 4, characterized in that the combination is present in a single composition, in which hydrocortisone, its salts or esters are present in proportions of between 0.01 and 3 % by weight relative to the total weight of the composition, preferably between 0.01 and 2 % and in particular between 0.01 and 1 %, and that the pyrimidine derivative of formula (I) is employed in proportions of between 0.05 and 6 % by weight relative to the total weight of the composition, preferably between 0.1 and 5 % and in particular between 0.5 and 3 % by weight.

6. Combination according to any one of Claims 1 to 5, characterized in that the anhydrous, physiologically acceptable medium for the components (A) and (B) is a medium consisting of a mixture of cosmetically or pharmaceutically acceptable organic solvents chosen from C₁-C₄ lower alcohols, alkylene glycols and mono- and dialkylene glycol alkyl ethers.

7. Combination according to any one of Claims 1 to 6, characterized in that at least one of the components (A) or (B) is thickened by means of thickening or gelling agents.

8. Combination according to any one of Claims 1 to 7, characterized in that at least one of the components (A) or (B) also contains cosmetically or pharmaceutically acceptable adjuvants chosen from preserving agents, complexing agents, colourants, alkalifying or acidifying agents, anionic, cationic, nonionic or amphoteric surface-active agents and mixtures thereof, and anionic, cationic, nonionic or amphoteric polymers, as well as mixtures thereof.

9. Multicompartment device or kit, characterized in that it comprises, in a first compartment, a component (A) such as defined in any one of Claims 1 to 8, and in a second compartment the component (B) such as also defined in any one of Claims 1 to 9.

10. Combination according to any one of Claims 1 to 8, for its application as a medication for the therapeutic treatment of hair loss.

11. Use of the combination according to any one of Claims 1 to 8 for the preparation of a medication intended to be employed in the therapeutic treatment of androgenetic alopecia.

## Claims (Claims for the following Contracting State(s): GR)

1. Combination intended to induce and to stimulate the growth of hair and to reduce its loss, characterized in that it comprises:
a) a component (A) containing, in an anhydrous, physiologically acceptable medium, at least hydrocortisone, its salts or esters, and
b) a component (B) containing, in an anhydrous physiologically acceptable medium, at least one pyrimidine derivative corresponding to the formula: 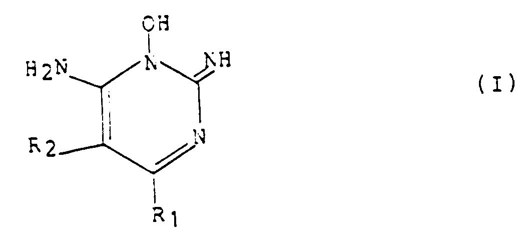 in which R₁ denotes a group 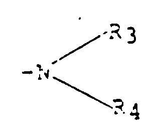 in which R₃ and R₄, independently of each other, denote hydrogen or an alkyl, alkenyl, alkylaryl or cycloalkyl group; R₃ and R₄ may also form a heterocyclic ring with the nitrogen atom to which they are linked, which is chosen from aziridinyl, azetidinyl, pyrrolidinyl, piperidyl, hexahydroazepinyl, heptamethyleneimine, octamethyleneimine, morpholine and 4-(lower)alkylpiperazidinyl groups, it being possible for the heterocyclic groups to be substituted on the carbon atoms by one to three lower alkyl, hydroxyl or alkoxy groups; the group R₂ is chosen from a hydrogen atom and an alkyl, alkenyl, alkylalkoxy, cycloalkyl, aryl, alkylaryl, arylalkyl, alkylarylalkyl, alkoxyarylalkyl or haloarylalkyl group, and the addition salts of physiologically acceptable acids, the components (A) and (B) forming part of the same single composition or being intended to be employed separately, either simultaneously or successively or after an interval of time on the scalp or the hair.

2. Combination according to Claim 1, characterized in that the hydrocortisone salts or esters are chosen from the 21-acetate, the 21-bendazac, the 17-valerate, the 17-butyrate, the 21-cyclopentylpropionate, the 21-hemisuccinate, the hexanoate, the disodium 21-phosphate, the sodium 21-succinate and the tert-butylacetate.

3. Combination according to Claims 1 or 2, characterized in that the pyrimidine derivative of formula (I) is 6-amino-1,2-dihydro-1-hydroxy-2-imino-4-piperidino-pyrimidine or "Minoxidil".

4. Combination according to any one of Claims 1 to 3, characterized in that hydrocortisone, its salts or esters are present in component (A) in proportions of between 0.01 and 5 % by weight, preferably between 0.02 and 3 % and in particular between 0.02 and 1 % by weight, and that the pyrimidine derivative is present in component (B) in proportions of between 0.05 and 10 % by weight, preferably between 0.05 and 5 % by weight and in particular between 0.5 and 4 % by weight.

5. Combination according to any one of Claims 1 to 4, characterized in that the combination is present in a single composition, in which hydrocortisone, its salts or esters are present in proportions of between 0.01 and 3 % by weight relative to the total weight of the composition, preferably between 0.01 and 2 % and in particular between 0.01 and 1 %, and that the pyrimidine derivative of formula (I) is employed in proportions of between 0.05 and 6 % by weight relative to the total weight of the composition, preferably between 0.1 and 5 % and in particular between 0.5 and 3 % by weight.

6. Combination according to any one of Claims 1 to 5, characterized in that the anhydrous, physiologically acceptable medium for the components (A) and (B) is a medium consisting of a mixture of cosmetically or pharmaceutically acceptable organic solvents chosen from C₁-C₄ lower alcohols, alkylene glycols and mono- and dialkylene glycol alkyl ethers.

7. Combination according to any one of Claims 1 to 6, characterized in that at least one of the components (A) or (B) is thickened by means of thickening or gelling agents.

8. Combination according to any one of Claims 1 to 7, characterized in that at least one of the components (A) or (B) also contains cosmetically or pharmaceutically acceptable adjuvants chosen from preserving agents, complexing agents, colourants, alkalifying or acidifying agents, anionic, cationic, nonionic or amphoteric surface-active agents and mixtures thereof, and anionic, cationic, nonionic or amphoteric polymers, as well as mixtures thereof.

9. Multicompartment device or kit, characterized in that it comprises, in a first compartment, a component (A) such as defined in any one of Claims 1 to 8, and in a second compartment the component (B) such as also defined in any one of Claims 1 to 9.

10. Process for the preparation of a combination consisting of two components (A) and (B) intended to induce and to stimulate the growth of hair and to reduce its loss, characterized in that
a) a component (A) is prepared by introducing into an anhydrous, physiologically acceptable medium, at least hydrocortisone, its salts or esters, and
b) a component (B) is prepared by introducing into an anhydrous physiologically acceptable medium, at least one pyrimidine derivative corresponding to the formula: 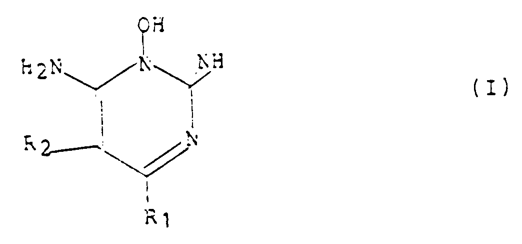 in which R₁ denotes a group 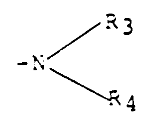 in which R₃ and R₄, independently of each other, denote hydrogen or an alkyl, alkenyl, alkylaryl or cycloalkyl group; R₃ and R₄ may also form a heterocyclic ring with the nitrogen atom to which they are linked, which is chosen from aziridinyl, azetidinyl, pyrrolidinyl, piperidyl, hexahydroazepinyl, heptamethyleneimine, octamethyleneimine, morpholine and 4-(lower)alkylpiperazidinyl groups, it being possible for the heterocyclic groups to be substituted on the carbon atoms by one to three lower alkyl, hydroxyl or alkoxy groups; the group R₂ is chosen from a hydrogen atom and an alkyl, alkenyl, alkylalkoxy, cycloalkyl, aryl, alkylaryl, arylalkyl, alkylarylalkyl, alkoxyarylalkyl or haloarylalkyl group, and the addition salts of physiologically acceptable acids,
the components (A) and (B) either being mixed or stored separately, with a view to being applied directly or successively or after an interval of time on the scalp or the hair.

## Patentansprüche (Patentansprüche für folgende(n) Vertragsstaat(en): AT, BE, CH, DE, FR, GB, IT, LI, NL, SE)

1. Assoziat zum Induzieren und Stimulieren des Wachstums der Haare, und zum Vermindern von deren Ausfall,
dadurch **gekennzeichnet**, daß
es umfasst:
a) einen Bestandteil (A), enthaltend, in einem wasserfreien physiologisch verträglichen wasserfreien Milieu, mindestens eine Hydrocortisonverbindung oder seine Salze und Ester, sowie
b) einen Bestandteil (B), enthaltend, in einem wasserfreien physiologisch verträglichen wasserfreien Milieu, mindestens ein Pyrimidinderivat der Formel: 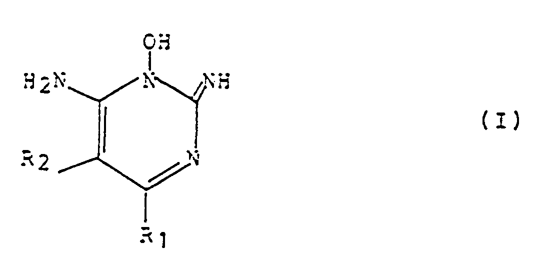 worin R1 eine 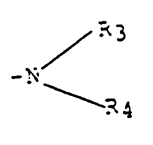 - Gruppe bedeutet,
worin R3 und R4, unabhängig voneinander, Wasserstoff, eine Alkyl-, Alkenyl-, Alkylaryl- und Cycloalkylgruppe bedeuten, wobei R3 und R4 auch einen Heterozyklus mit dem Stickstoffatom bilden können, an das sie gebunden sind, ausgewählt aus Aziridinyl-, Azetidinyl-, Pyrrolidinyl-, Piperidinyl-, Hexahydroazepinyl-, Heptamethylenimin-, Octamethylenimin-, Morpholin- und 4-(niedrig)-Akylpiperazidinylgruppen, wobei die heterozyklischen Gruppen an den Kohlenstoffatomen mit 1 bis 3 Niedrigalkyl-, Hydroxy- oder Alkoxygruppen substituiert sein können; und worin die Gruppe R2 ausgewählt ist aus Wasserstoff, einer Alkyl-, Akenyl-, Alkylalkoxy-, Cycloalkyl-, Aryl-, Alkylaryl-, Arylakyl-, Alkylarylalkyl-, Alkoxyarylalkyl- oder Haloarylalkylgruppe, sowie die physiologisch verträglichen Säureadditionssalze davon, wobei die Bestandteile (A) und (B) in derselben Zusammensetzung vorliegen oder auf der behaarten Haut oder den Haaren getrennt zu verwenden sind, entweder gleichzeitig oder nacheinander oder nach einem Zeitablauf.

2. Assoziat nach Anspruch 1,
dadurch **gekennzeichnet**, daß
die Salze oder Ester von Hydrocortison sind ausgewählt insbesondere aus dem 21-Acetat, 21-Bendazac, 17-Valerat, 17-Butyrat, 21-Cyclopentylpropionat, 21-Hemisuccinat, Hexanoat, Dinatrium-21-phosphat, Natrium-21-succinat, t-Butylacetat.

3. Assoziat gemäß Ansprüchen 1 oder 2,
dadurch **gekennzeichnet**, daß
das Pyrimidinderivat der Formel (I) 6-Amino-1,2-dihydro-1-hydroxy-2-imino-4-piperidinopyrimidin, auch bezeichnet als "Minoxidil", ist.

4. Assoziat gemäß jedem Anspruch 1 bis 3,
dadurch **gekennzeichnet**, daß
das Hydrocortison, seine Salze oder Ester im Bestandteil (A) in Mengenverhältnissen von 0,01 bis 5, vorzugsweise 0,02 bis 3 und insbesondere 0,02 bis 1, Gew.% und das Pyrimidinderivat im Bestandteil (B) in Mengenverhältnissen von 0,05 bis 10, vorzugsweise 0,05 bis 5 und insbesondere 0,05 bis 4, Gew.% vorliegen.

5. Assoziat gemäß jedem Anspruch 1 bis 4,
dadurch **gekennzeichnet**, daß
das Assoziat in einer einzigen Zusammensetzung vorliegt, worin das Hydrocortison, seine Salze oder Ester in Mengenverhältnissen von 0,01 bis 3, vorzugsweise 0,01 bis 2 und insbesondere 0,01 bis 1, Gew.%, bezogen auf Gesamtgewicht der Zusammensetzung, und das Pyrimidinderivat der Formel (I) in Mengenverhältnissen von 0,05 bis 6, vorzugsweise 0,1 bis 5 und insbesondere 0,5 bis 3, Gew.%, bezogen auf Gesamtgewicht der Zusammensetzung, vorhanden sind.

6. Assoziat gemäß jedem Anspruch 1 bis 5,
dadurch **gekennzeichnet**, daß
das physiologisch verträgliche wasserfreie Milieu für die Bestandteile (A) und (B) ein Milieu aus einer Mischung von in Kosmetik oder Pharmazie zulässigen organischen Lösungsmitteln ist, ausgewählt aus C₁₋₄-Niedrigalkoholen, Alkylenglycolen, Alkylethern von Mono- und Dialkylenglycol.

7. Assoziat gemäß jedem Anspruch 1 bis 6,
dadurch **gekennzeichnet**, daß
mindestens einer der Bestandteile (A) und (B) mit verdickenden oder gelierenden Mitteln verdickt ist.

8. Assoziat gemäß jedem Anspruch 1 bis 7,
dadurch **gekennzeichnet**, daß
mindestens einer der Bestandteile (A) oder (B) auch kosmetisch oder pharmazeutisch verträgliche Hilfstoffe enthält, ausgewählt aus Konservierungs-, Komplexier-, Färbe-, alkalisch oder sauermachenden, aus anionischen, kationischen, nicht-ionischen oder amphoteren oberflächenaktiven Mitteln, sowie deren Mischungen, und aus anionischen, kationischen, nicht-ionischen oder amphoteren Polymeren, sowie deren Mischungen.

9. Vorrichtung aus mehreren Teilen oder "Kit",
dadurch **gekennzeichnet**, daß
sie in einem ersten Teil einen in jedem der Ansprüche 1 bis 8 definierten Bestandteil (A) und in einem zweiten Teil den ebenso in jedem der Ansprüche 1 bis 8 definierten Bestandteil (B) enthalten.

10. Assoziat gemäß jedem Anspruch 1 bis 8
zur Aufbringung als Medikament zur therapeutischen Behandlung des Ausfalls von Haaren.

11. Verwendung des Assoziats gemäß jedem Anspruch 1 bis 8
zur Herstellung eines Medikaments zur Verwendung bei der therapeutischen Behandlung androgenetischer Alopezie.

## Patentansprüche (Patentansprüche für folgende(n) Vertragsstaat(en): GR)

1. Assoziat zum Induzieren und Stimulieren des Wachstums der Haare, und zum Vermindern von deren Ausfall,
dadurch **gekennzeichnet**, daß
es umfasst:
a) einen Bestandteil (A), enthaltend, in einem wasserfreien physiologisch verträglichen wasserfreien Milieu, mindestens eine Hydrocortisonverbindung oder seine Salze und Ester, sowie
b) einem Bestandteil (B), enthaltend, in einem wasserfreien physiologisch verträglichen wasserfreien Milieu, mindestens ein Pyrimidinderivat der Formel: 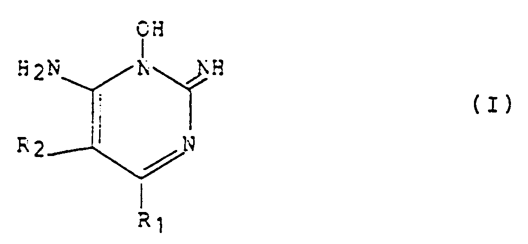 worin R1 eine 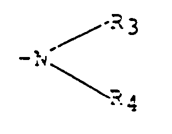 - Gruppe bedeutet,
worin R3 und R4, unabhängig voneinander, Wasserstoff, eine Alkyl-, Alkenyl-, Alkylaryl- und Cycloalkylgruppe bedeuten, wobei R3 und R4 auch einen Heterozyklus mit dem Stickstoffatom bilden können, an das sie gebunden sind, ausgewählt aus Aziridinyl-, Azetidinyl-, Pyrrolidinyl-, Piperidinyl-, Hexahydroazepinyl-, Heptamethylenimin-, Octamethylenimin-, Morpholin- und 4-(niedrig)-Akylpiperazidinylgruppen, wobei die heterozyklischen Gruppen an den Kohlenstoffatomen mit 1 bis 3 Niedrigalkyl-, Hydroxy- oder Alkoxygruppen substituiert sein können; und worin die Gruppe R2 ausgewählt ist aus Wasserstoff, einer Alkyl-, Akenyl-, Alkylalkoxy-, Cycloalkyl-, Aryl-, Alkylaryl-, Arylakyl-, Alkylarylalkyl-, Alkoxyarylalkyl- oder Haloarylalkylgruppe, sowie die physiologisch verträglichen Säureadditionssalze davon, wobei die Bestandteile (A) und (B) in derselben Zusammensetzung vorliegen oder auf der behaarten Haut oder den Haaren getrennt zu verwenden sind, entweder gleichzeitig oder nacheinander oder nach einem Zeitablauf.

2. Assoziat nach Anspruch 1,
dadurch **gekennzeichnet**, daß
die Salze oder Ester von Hydrocortison sind ausgewählt insbesondere aus dem 21-Acetat, 21-Bendazac, 17-Valerat, 17-Butyrat, 21-Cyclopentylpropionat, 21-Hemisuccinat, Hexanoat, Dinatrium-21-phosphat, Natrium-21-succinat, t-Butylacetat.

3. Assoziat gemäß Ansprüchen 1 oder 2,
dadurch **gekennzeichnet**, daß
das Pyrimidinderivat der Formel (I) 6-Amino-1,2-dihydro-1-hydroxy-2-imino-4-piperidinopyrimidin, auch bezeichnet als "Minoxidil", ist.

4. Assoziat gemäß jedem Anspruch 1 bis 3,
dadurch **gekennzeichnet**, daß
das Hydrocortison, seine Salze oder Ester im Bestandteil (A) in Mengenverhältnissen von 0,01 bis 5, vorzugsweise 0,02 bis 3 und insbesondere 0,02 bis 1, Gew.% und das Pyrimidinderivat im Bestandteil (B) in Mengenverhältnissen von 0,05 bis 10, vorzugsweise 0,05 bis 5 und insbesondere 0,05 bis 4, Gew.% vorliegen.

5. Assoziat gemäß jedem Anspruch 1 bis 4,
dadurch **gekennzeichnet**, daß
das Assoziat in einer einzigen Zusammensetzung vorliegt, worin das Hydrocortison, seine Salze oder Ester in Mengenverhältnissen von 0,01 bis 3, vorzugsweise 0,01 bis 2 und insbesondere 0,01 bis 1, Gew.%, bezogen auf Gesamtgewicht der Zusammensetzung, und das Pyrimidinderivat der Formel (I) in Mengenverhältnissen von 0,05 bis 6, vorzugsweise 0,1 bis 5 und insbesondere 0,5 bis 3, Gew.%, bezogen auf Gesamtgewicht der Zusammensetzung, vorhanden sind.

6. Assoziat gemäß jedem Anspruch 1 bis 5,
dadurch **gekennzeichnet**, daß
das physiologisch verträgliche wasserfreie Milieu für die Bestandteile (A) und (B) ein Milieu aus einer Mischung von in Kosmetik oder Pharmazie zulässigen organischen Lösungsmitteln ist, ausgewählt aus C₁₋₄-Niedrigalkoholen, Alkylenglycolen, Alkylethern von Mono- und Dialkylenglycol.

7. Assoziat gemäß jedem Anspruch 1 bis 6,
dadurch **gekennzeichnet**, daß
mindestens einer der Bestandteile (A) und (B) mit verdickenden oder gelierenden Mitteln verdickt ist.

8. Assoziat gemäß jedem Anspruch 1 bis 7,
dadurch **gekennzeichnet**, daß
mindestens einer der Bestandteile (A) oder (B) auch kosmetisch oder pharmazeutisch verträgliche Hilfstoffe enthält, ausgewählt aus Konservierungs-, Komplexier-, Färbe-, alkalisch oder sauermachenden, das anionischen, kationischen, nicht-ionischen oder amphoteren oberflächenaktiven Mitteln, sowie deren Mischungen, und aus anionischen, kationischen, nicht-ionischen oder amphoteren Polymeren, sowie deren Mischungen.

9. Vorrichtung aus mehreren Teilen oder "Kit",
dadurch **gekennzeichnet**, daß
sie in einem ersten Teil einen in jedem der Ansprüche 1 bis 8 definierten Bestandteil (A) und in einem zweiten Teil den ebenso in jedem der Ansprüche 1 bis 8 definierten Bestandteil (B) enthalten.

10. Verfahren zur Herstellung eines Assoziats aus 2 Bestandteilen (A) und (B) zum Induzieren und Stimulieren des Wachstums der Haare und zum Vermindern von deren Ausfall,
dadurch **gekennzeichnet**, daß
man
a) einen Bestandteil (A) herstellt, indem man in ein physiologisch verträgliches wasserfreies Milieu mindestens eine Hydrocortisonverbindung, seine Salze oder Ester einbringt, und
b) einen Bestandteil (B) herstellt, indem man in ein physiologisch verträgliches wasserfreies Milieu mindestens ein Pyrimidinderivat der Formel einbringt: 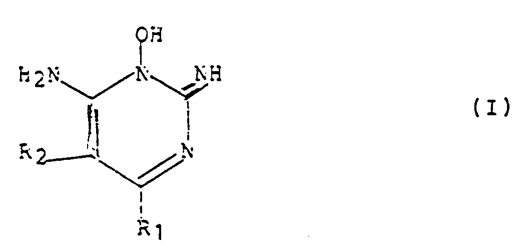 worin R1 eine 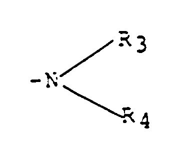 - Gruppe bedeutet,
worin R3 und R4, unabhängig voneinander, Wasserstoff, eine Alkyl-, Alkenyl-, Alkylaryl- und Cycloalkylgruppe bedeuten, wobei R3 und R4 auch einen Heterozyklus mit dem Stickstoffatom bilden können, an das sie gebunden sind, ausgewählt aus Aziridinyl-, Azetidinyl-, Pyrrolidinyl-, Piperidinyl-, Hexahydroazepinyl-, Heptamethylenimin-, Octamethylenimin-, Morpholin- und 4-(niedrig)-Akylpiperazidinylgruppen, wobei die heterozyklischen Gruppen an den Kohlenstoffatomen mit 1 bis 3 Niedrigalkyl-, Hydroxy- oder Alkoxygruppen substituiert sein können; und worin die Gruppe R2 ausgewählt ist aus Wasserstoff, einer Alkyl-, Akenyl-, Alkylalkoxy-, Cycloalkyl-, Aryl-, Alkylaryl-, Arylakyl-, Alkylarylalkyl-, Alkoxyarylalkyl- oder Haloarylalkylgruppe, sowie die physiologisch verträglichen Säureadditionssalze davon,
wobei die Bestandteile (A) und (B) entweder vermischt oder getrennt aufbewahrt vorliegen, und zwar im Hinblick auf deren Aufbringung auf behaarte Haut oder Haare, sei es direkt oder nacheinander oder nach einem Zeitablauf.
